# EUROPEAN PATENT APPLICATION

(11) **EP 3 913 021 A1**
(43) Date of publication of application: **24.11.2021**
(21) Application number: 19910126.2
(22) Date of filing: 12.12.2019
(51) Int. Cl.: C08L 77/00, C08L 75/04, C08L 53/00, C08L 23/08, C08L 53/02, C08K 9/04, C08K 3/34

(54) **MEDICAL MATERIAL AND PREPARATION METHOD THEREFOR**

(30) Priority: 14.01.2019 CN 201910033433
(71) Applicant: Accupath Medical (Jiaxing) Co., Ltd., Jiaxing, Zhejiang 314006 (CN)
(72) Inventor: LI, Zhaomin, Jiaxing, Zhejiang 314006 (CN); QIN, Minglin, Jiaxing, Zhejiang 314006 (CN); HE, Guangbin, Jiaxing, Zhejiang 314006 (CN); DENG, Zhihua, Jiaxing, Zhejiang 314006 (CN); SUN, Quanquan, Jiaxing, Zhejiang 314006 (CN); LI, Ruipei, Jiaxing, Zhejiang 314006 (CN); LIU, Yifan, Jiaxing, Zhejiang 314006 (CN); QUE, Yiyun, Jiaxing, Zhejiang 314006 (CN)
(74) Representative: Dauncey, Mark Peter
(86) International application number: PCT/CN2019/124946
(87) International publication number: WO 2020/147470

(57) **Abstract**

Disclosed are a medical material and a preparation method therefor. The preparation method comprises the following steps: S1: adding a nano montmorillonite into a solution of a silane coupling agent, followed by stirring, rinsing, and drying to obtain a modified nano montmorillonite; and S2: mixing a thermoplastic elastomer with the modified nano montmorillonite to obtain the medical material. The medical material prepared according to the present invention improves the modulus, breaking strength and flexural modulus of the thermoplastic elastomer, and broadens the application of the thermoplastic elastomer in the field of high-end minimally invasive interventional medical products; simplifies a production process; and increases productivity. The prepared medical material is intended for use in a delivery device so as to enhance the push and torque performance of the delivery device, such that the delivery device can be applied in a medical tubing product, including a guide catheter, a drug stent delivery device, a dilation balloon catheter, a contrast catheter, or an electrophysiology catheter, etc.

## Description

### TECHNICAL FIELD

The present disclosure relates to a biomedical raw material and a method for preparing the same, in particular to a medical material, a medical tube and a method for preparing the same.

### BACKGROUND

Minimally invasive interventional medical technology is one of the important contributions of medicine to human civilization at the end of the 20th century, covering science and technology in cardiovascular, cerebrovascular, aortic, peripheral, electrophysiological and other fields. Micro-sized medical tubes, as a key material for minimally invasive interventional medical devices, are very strictly demanded by the minimally invasive interventional medical devices and have great technical difficulties. They have always been monopolized by European and American companies. In the past 20 years, the main technical difficulties in the industrialization of micro-sized medical tubes for minimally invasive interventional medical devices in China involve key technologies such as synthesis of medical polymer materials, modification of medical polymer materials, micro-extrusion, welding, grinding, weaving, springs, and regulation of condensed matter structures, and key equipment for the same.

### SUMMARY

### TECHNICAL PROBLEMS

The same series of medical polymer materials with different hardnesses have good compatibility and thermal meltability. When used in minimally invasive interventional medical devices, different materials can be selected for different parts of a catheter according to the needs, so as to obtain a catheter with gradual hardness. Because catheters and conveyors of the minimally invasive interventional medical devices are usually relatively long, the front sections thereof are required to be soft so as to be able to pass through the curved blood vessels in the human body to reach the lesion; while the back sections thereof are required to have a certain degree of hardness to ensure sufficient support force during the catheter entering the human body. A transition section between each parts is required to have sufficient connection strength and smoothness. The connection strength ensures that the catheter will not be broken during use. The smooth transition section can reduce the damage of the catheter to the blood vessel, and passing resistance.

Existing thermoplastic elastomer materials have insufficient strength, resulting in that the catheter is easy to be bent when passing, affecting the passing performance thereof. Therefore, it is necessary to modify the existing raw materials to improve the passing performance of the catheter.

### SOLUTIONS TO PROBLEMS

### TECHNICAL SOLUTIONS

The technical problem to be solved by the present disclosure is to provide a medical material and a method for prepare the same, which can solve the problems of excessive toughness, insufficient rigidity and insufficient smoothness of the existing medical tubes made of thermoplastic elastomer, and improve the passing performance of catheters.

A technical solution to solve the above-mentioned technical problems adopted by the present disclosure is to provide a method for preparing a medical material, the method comprising steps of: S1, adding nano-montmorillonite to a solution of a silane coupling agent, followed by stirring, washing, and drying to obtain a modified nano-montmorillonite; and S2, mixing a thermoplastic elastomer with the modified nano-montmorillonite, followed by stirring to obtain the medical material.

Preferably, the silane coupling agent in step S1 is at least one of γ-aminopropyltriethoxysilane, γ-(2,3-glycidoxy)propyltrimethoxysilane or γ-(methacryloxy)propyltrimethoxysilane.

Preferably, the nano-montmorillonite in step S1 has a particle size of 1 nm -100 nm, and a volume density of 0.24 gms/cc-0.32 gms/cc.

Preferably, step S1 is performed in a process as follows: adding the nano-montmorillonite to the solution of the silane coupling agent, followed by stirring at a constant temperature of 50°C-70°C; washing and drying a resultant which is obtained after adding the nano-montmorillonite, to obtain the modified nano-montmorillonite.

Preferably, the thermoplastic elastomer is at least one of polyether block amide, thermoplastic polyurethane, styrene block copolymer, styrene-butadiene-styrene block copolymer or octene copolymer.

Preferably, in step S2, the modified nano-montmorillonite and the thermoplastic elastomer are in a mass ratio of 1%-5%.

Preferably, step S2 comprises: adding the thermoplastic elastomer and the modified nano-montmorillonite into a mixer for uniform mixing to obtain a mixture, and extruding the mixture with an extruder.

Preferably, the method further comprises a step of: S3, washing and condensing the extruded mixture, followed by granulating and drying.

Preferably, the extruding the mixture with the extruder includes: extruding the mixture with a co-rotating twin-screw extruder. The co-rotating twin-screw extruder has an aspect ratio of a length to a diameter of a screw, of 1:35 to 1:55, runs at a screw speed of 150 rpm to 1000 rpm, and has an extrusion temperature of 180°C to 230°C, an extruder head pressure of 3 MPa to 5 MPa, and a vacuum pump pressure is 0.8 MPa to 1 MPa.

Another technical solution to solve the above-mentioned technical problems adopted by the present disclosure is to provide a medical material that is a blend of a thermoplastic elastomer and nano-montmorillonite. The thermoplastic elastomer has a lamella structure, and forms a nano-crystal three-dimensional network structure with the nano-montmorillonite.

Preferably, the nano-montmorillonite and the thermoplastic elastomer in the blend of the thermoplastic elastomer and the nano-montmorillonite are in a mass ratio of 1%-5%.

Preferably, the thermoplastic elastomer is at least one of polyether block amide, thermoplastic polyurethane, styrene block copolymer, styrene-butadiene-styrene block copolymer or octene copolymer.

Preferably, the nano-montmorillonite is nano-montmorillonite modified with a silane coupling agent.

Compared with the prior art, the present disclosure has beneficial effects as follows. For the medical material and the method for preparing the same provided in the present disclosure, the nano-montmorillonite is used to modify the thermoplastic elastomer for an enhancement. Compared with ordinary montmorillonite, the nano-montmorillonite has a larger specific surface area. According to the "nano-crystal three-dimensional network structure model", the prepared thermoplastic elastomer/nano-montmorillonite blend can improve the modulus, fracture strength and flexural modulus of the thermoplastic elastomer, improve the mechanical properties to reach the level of similar products in developed countries, and expand the application of the thermoplastic elastomer in the field of high-end minimally invasive interventional medical products. In addition, the production process can be simplified, and production capacity can be increased. In particular, surface modification treatment of the nano-montmorillonite by adding the silane coupling agent can increase the compatibility and bonding force of the nano-montmorillonite with medical polymer materials. The prepared material is applied to a conveyor to improve the delivery and torque performance of the conveyor, so that the conveyor can be used in guiding catheters, drug stent conveyors, dilatation balloon catheters, angiographic catheters, electrophysiological catheters and other medical tube products.

### DETAILED DESCRIPTION OF THE EMBODIMENTS

### EXAMPLES

The present disclosure will be further described below in conjunction with examples.

### Example 1

First, a predetermined amount of a silane coupling agent was added to a prepared mixing solution of absolute ethanol, deionized water and glacial acetic acid, followed by stirring at room temperature for 2-6 hours. Specifically, the silane coupling agent may be KH550 silane coupling agent (γ-aminopropyltriethoxysilane), KH560 silane coupling agent (γ-(2,3-glycidoxy)propyltrimethoxysilane), KH570 silane coupling agent (γ-(methacryloxy)propyltrimethoxysilane) or the like.

Next, a predetermined amount of nano-montmorillonite was added to the above solution with further stirring at a constant temperature of 50°C-70°C for 8-24 hours. The resultant was washed three times with absolute ethanol, and then dried in a vacuum drying oven to obtain a modified nano-montmorillonite.

Then, dry polyether block amide (PEBA) and the modified nano-montmorillonite were added into a high-speed mixer according to a predetermined mass ratio and mixed uniformly. The nano-montmorillonite has a particle size of 15 nm, and a volume density of 0.24 gms/cc. The PEBA and the modified nano-montmorillonite is in a mass ratio of 100:1. Then, the mixture is extruded by a co-rotating twin-screw extruder. Temperatures of processes during the operating of the extruder are 190°C, 200°C, 210°C, 220°C, repectively. A screw speed is 150 rpm, an extruder head pressure is 3 MPa, and a vacuum pump pressure is 0.8 MPa. In other embodiments, the PEBA can also be replaced by thermoplastic polyurethane elastomer (TPU), styrene block copolymer (SBC), styrene-butadiene-styrene block copolymer (SBS) or octene copolymer (POE) and other thermoplastic elastomer materials.

Finally, the samples extruded by the co-rotating twin-screw extruder were washed and condensed by water in a water tank, then granulated and dried to obtain a medical material enhanced by the nano-montmorillonite.

### Example 2

The production process of this example was the same as that of Example 1, except that the PEBA and the modified nano-montmorillonite were in a different mass ratio, which is 100:5.

The nano-montmorillonite is selected in the present disclosure. Compared with ordinary montmorillonite, the nano-montmorillonite has a larger specific surface area. The well-dispersed nano-montmorillonite improves the modulus, fracture strength and flexural modulus of medical polymer materials.

In the present disclosure, surface modification treatment of the nano-montmorillonite by adding a silane coupling agent such as KH550, KH560 or KH570 increases the compatibility and bonding force of the nano-montmorillonite with the medical polymer materials. The montmorillonite has poor dispersion and compatibility in thermoplastic elastomers, however, hydrophobical modification by using the silane coupling agent can not only improve its lipophilicity, but also enhance its reactivity, allowing it to more easily chemically react with other functional groups, thereby improving the compatibility with other materials.

Therefore, in the present disclosure, the thermoplastic elastomer is blended with the nano-montmorillonite, and thus is modified by the nano-montmorillonite. The nano-montmorillonite is exfoliated into nano-scale structural sheets in the process of being blended with the thermoplastic elastomer melt, and is uniformly dispersed into the thermoplastic elastomer matrix. In the subsequent extrusion process, under the action of high-stretching, the thermoplastic elastomer spherulites in the blend are transformed into lamellae. With the lamella structure of the nano-montmorillonite, a nano-crystal three-dimensional network structure is formed from the thermoplastic elastomer and the nano-montmorillonite, thereby significantly improving the modulus, fracture strength and flexural modulus of the thermoplastic elastomer. Through the modification and research of this kind of material, it is applied to a conveyor to improve the delivery and torque performance of the conveyor, so that the conveyor can be used in guiding catheters, drug stent conveyors, dilatation balloon catheters, angiographic catheters, electrophysiological catheters and other products.

Although the present disclosure has been disclosed as above in preferred embodiments, they are not intended to limit the present disclosure. Some modifications and improvements can be made by one skilled in the art without departing from the spirit and scope of the present disclosure. Thus, the protection scope of the present disclosure should be defined by the claims.

## Claims

1. A method for preparing a medical material, comprising steps of:
S1, adding nano-montmorillonite to a solution of a silane coupling agent, followed by stirring, washing, and drying to obtain a modified nano-montmorillonite; and
S2, mixing a thermoplastic elastomer with the modified nano-montmorillonite, followed by stirring to obtain the medical material.

2. The method according to claim 1, wherein the silane coupling agent in the step S1 is at least one of γ-aminopropyltriethoxysilane, γ-(2,3-glycidoxy)propyltrimethoxysilane or γ-(methacryloxy)propyltrimethoxysilane.

3. The method according to claim 1, wherein the nano-montmorillonite in the step S1 has a particle size of 1 nm-100 nm, and a volume density of 0.24 gms/cc-0.32 gms/cc.

4. The method according to claim 1, wherein the step S1 comprises: adding the nano-montmorillonite to the solution of the silane coupling agent, followed by stirring at a constant temperature of 50°C-70°C; washing and drying a resultant which is obtained after adding the nano-montmorillonite, to obtain the modified nano-montmorillonite.

5. The method according to claim 1, wherein the thermoplastic elastomer is at least one of polyether block amide, thermoplastic polyurethane, styrene block copolymer, styrene-butadiene-styrene block copolymer or octene copolymer.

6. The method according to claim 1, wherein in the step S2, the modified nano-montmorillonite and the thermoplastic elastomer are in a mass ratio of 1%-5%.

7. The method according to claim 1, wherein the step S2 comprises: adding the thermoplastic elastomer and the modified nano-montmorillonite into a mixer for uniform mixing to obtain a mixture, and extruding the mixture with an extruder.

8. The method according to claim 7, further comprising a step of: S3, washing and condensing the extruded mixture, followed by granulating and drying.

9. The method according to claim 7, wherein the extruding the mixture with the extruder comprises: extruding the mixture with a co-rotating twin-screw extruder, wherein the co-rotating twin-screw extruder has an aspect ratio of a length to a diameter of a screw, of 1:35 to 1:55, runs at a screw speed of 150 rpm to 1000 rpm, and has an extrusion temperature of 180°C to 230°C, an extruder head pressure of 3 MPa to 5 MPa, and a vacuum pump pressure of 0.8 MPa to 1 MPa.

10. A medical material, wherein the medical material is a blend of a thermoplastic elastomer and nano-montmorillonite, the thermoplastic elastomer has a lamella structure and forms a nano-crystal three-dimensional network structure with the nano-montmorillonite.

11. The medical material according to claim 10, wherein the nano-montmorillonite and the thermoplastic elastomer in the blend of the thermoplastic elastomer and the nano-montmorillonite are in a mass ratio of 1%-5%.

12. The medical material according to claim 10, wherein the thermoplastic elastomer is at least one of polyether block amide, thermoplastic polyurethane, styrene block copolymer, styrene-butadiene-styrene block copolymer or octene copolymer.

13. The medical material according to claim 10, wherein the nano-montmorillonite is nano-montmorillonite modified with a silane coupling agent.
